Europäisches Patentamt

European Patent Office

Office européen des brevets

(11) Numéro de publication: **0 214 057**

**A1**

(12) # DEMANDE DE BREVET EUROPEEN

(21) Numéro de dépôt: **86401916.1**

(22) Date de dépôt: **01.09.86**

(51) Int. Cl.⁴: **A 61 K 37/02**
**A 61 K 39/095, A 61 K 39/40**
**G 01 N 33/571, G 01 N 33/5-**
**48**
**G 01 N 33/561**

(30) Priorité: **03.09.85 FR 8513066**

(43) Date de publication de la demande:
**11.03.87 Bulletin 87/11**

(84) Etats contractants désignés:
**AT BE CH DE FR GB IT LI LU NL SE**

(71) Demandeur: **INSTITUT PASTEUR DE LYON ET DU SUD-EST**
**77, rue Pasteur**
**F-69007 Lyon(FR)**

(71) Demandeur: **Guinet, Roland**
**"Au Charmillon" Sainte Consorce**
**F-69260 Charbonnières les Bains(FR)**

(71) Demandeur: **Perrollet, Hervé**
**21, rue Edison**
**F-69003 Lyon(FR)**

(72) Inventeur: **Guinet, Roland**
**Au Charmillion-Sainte Consorce**
**F-69260 Charbonnieres Les Bains(FR)**

(72) Inventeur: **Perrollet, Hervé**
**21, rue Edison**
**F-69003 Lyon(FR)**

(74) Mandataire: **Warcoin, Jacques et al,**
**Cabinet Régimbeau 26, avenue Kléber**
**F-75116 Paris(FR)**

(54) **Lectines-facteurs d'attachement de Neisseria gonorrhoeae, procédé pour leur préparation et composition thérapeutique contenant ces lectines ou les anticorps correspondants ou les récepteurs d'attachement correspondants.**

(57) L'invention concerne un procédé de préparation de lectines-facteurs d'attachement de Neisseria gonorrhoeae, caractérisé en ce que :

a) on prépare à partir d'une souche de Neisseria gonorrhoeae un extrait antigénique pariétal solubilisé ultérieurement,

b) on met cet extrait en présence d'un récepteur sucré immobilisé,

c) puis on sépare les lectines-facteurs d'attachement fixées sur ledit récepteur.

## LECTINES-FACTEURS D'ATTACHEMENT DE NEISSERIA GONORRHOEAE, PROCEDE POUR LEUR PREPARATION ET COMPOSITION THERAPEUTIQUE CONTENANT CES LECTINES OU LES ANTICORPS CORRESPONDANTS OU LES RECEPTEURS D'ATTACHEMENT CORRESPONDANTS

La présente invention concerne des lectines-facteurs d'attachement de Neisseria gonorrhoeae ainsi que l'application de certains récepteurs immobilisés pour la préparation de ces facteurs et leur mise en évidence ou leur dosage.

Plus particulièrement, il s'agit de la mise en évidence et de la caractérisation de lectines-facteurs d'attachement chez Neisseria gonorrhoeae d'origines diverses : souches de différentes collections de référence ainsi que souches responsables de gonococcies localisées et souches responsables de gonococcies disséminées.

Ces facteurs d'attachement de Neisseria gonorrhoeae du type lectine ont pu être mis en évidence grâce à leur propriété d'adhésion à des récepteurs immobilisés sucrés, en particulier des récepteurs de type glucosamine ou maltose.

La fixation des sucres précédents sur des supports immobilisants tels que l'agarose permet de purifier les lectines-facteurs d'attachement. Ces lectines-facteurs d'attachement, en raison de leur propriété antigénique, et/ou de leur activité de type lectine, peuvent être utilisées dans des compositions pharmaceutiques, elles-mêmes ou les anticorps correspondants, soit à titre curatif, soit à titre préventif, pour le traitement ou surtout la prévention des infections gonococciques et également des infections mixtes avec d'autres bactéries, anaérobies par exemple.

Ces lectines-facteurs d'attachement sont particulièrement intéressantes étant donné la recrudescence mondiale actuelle de la gonococcie et de ses forme asymptomatiques. Ces lectines-facteurs d'attachement pourront entraîner une rupture de la chaîne épidémiologique soit par l'établissement d'un diagnostic de laboratoire, soit, et surtout, par la mise au point d'un produit pharmaceutique préventif.

La présente invention concerne également un procédé de préparation de ces lectines-facteurs d'attachement de Neisseria gonorrhoeae, caractérisé en ce que :

a) des extraits protéiques de la membrane externe de Neisseria gonorrhoeae sont préparés puis solubilisés,

b) ces extraits sont mis en présence de récepteurs sucrés immobilisés,

c) puis les lectines-facteurs d'attachement ainsi fixées aux récepteurs sont séparés.

Parmi les récepteurs sucrés immobilisés utilisables dans le cadre de ce procédé, il faut citer plus particulièrement les récepteurs sucrés immobilisés sur support agarose de type glucosamine, maltose. Les récepteurs immobilisés peuvent se présenter sous une forme quelconque; en particulier, ils peuvent être utilisés sous forme de colonne ou bien mis en oeuvre par "batch".

Enfin, les propriétés de fixation de ces lectines-facteurs d'attachement et leur spécificité permettent d'envisager des procédés de dosage ou de mise en évidence de Neisseria gonorrhoeae notamment dans les produits pathologiques.

Ces procédés de mise en évidence de Neisseria gonorrhoeae comporteront au moins une étape de fixation des lectines-facteurs d'attachement de Neisseria gonorrhoeae sur un sucre récepteur de types glucosamine ou maltose, ce sucre récepteur étant de préférence immobilisé sur un support tel qu'un support d'agarose.

Les lectines-facteurs d'attachement présentant des propriétés antigéniques, il est possible de les mettre en évidence et/ou de les doser par des procédés immunologiques, en particulier par des procédés d'immunoélectrophorèse et immunoenzymatique.

Dans le cadre d'un dosage de type immunologique, on pourra doser ou mettre en évidence la présence de Neisseria gonorrhoeae dans un prélèvement en recherchant la disparition du précipité caractéristique antigène/anticorps dans le prélèvement soumis préalablement à un traitement afin de libérer ces lectines-facteurs d'attachement de la membrane externe du gonocoque et à l'action d'un sucre récepteur fixant lesdites lectines-facteurs d'attachement.

Il est également possible de metre en évidence les précipités spécifiques antigènes/anticorps correspondant aux lectines-facteurs d'attachement à partir du prélèvement ayant été soumis au traitement d'extraction de ces facteurs pariétaux puis ayant été traités par des récepteurs sucrés immobilisés, lesdits récepteurs sucrés immobilisés étant séparés puis remis en solution pour libérer les éventuelles lectines-facteurs d'attachement.

Bien entendu, la présence ou au contraire l'absence de ces lectines-facteurs d'attachement caractérisées par des méthodes immunologiques mettant en oeuvre les réactions antigènes/anticorps peuvent être de types très variés, il peut en particulier s'agir d'immunoélectrophorèse telle que l'immunoélectrophorèse de type "line-absorption" ou toute autre méthode immunologique bien connue de l'homme de métier.

Enfin, il est possible d'élaborer des anticorps contre les antigènes des lectines-facteurs d'attachement par des procédés connus, lesdits anticorps étant également utiles à titre de composants thérapeutiques et de réactifs pour le diagnostic.

Les exemples suivants sont destinés à mettre en évidence d'autres caractéristiques et avantages de la présente invention.

-4-                                    0214057

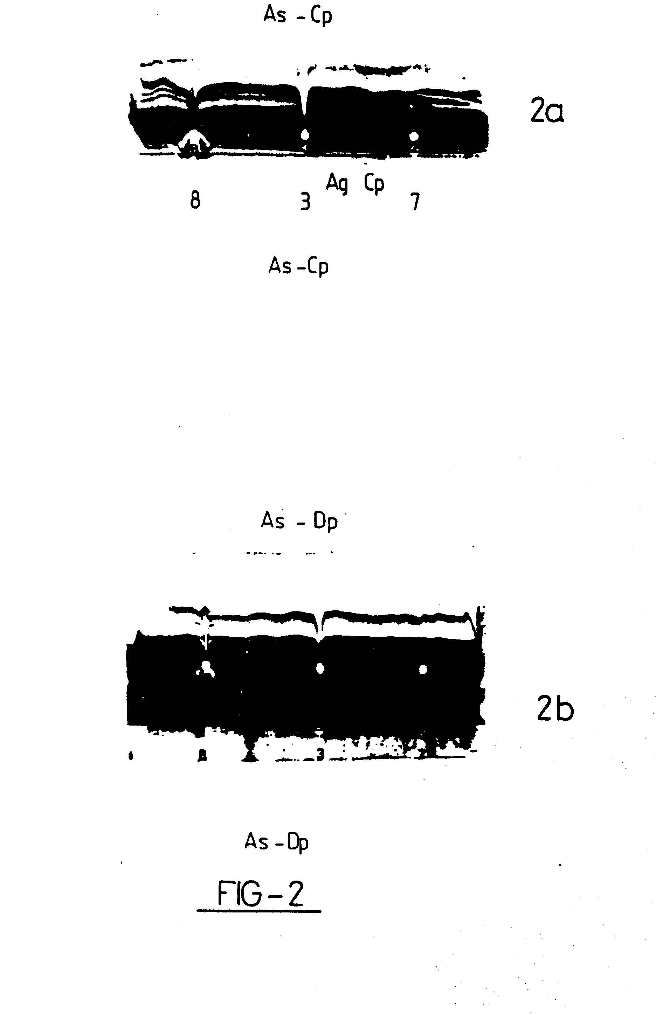

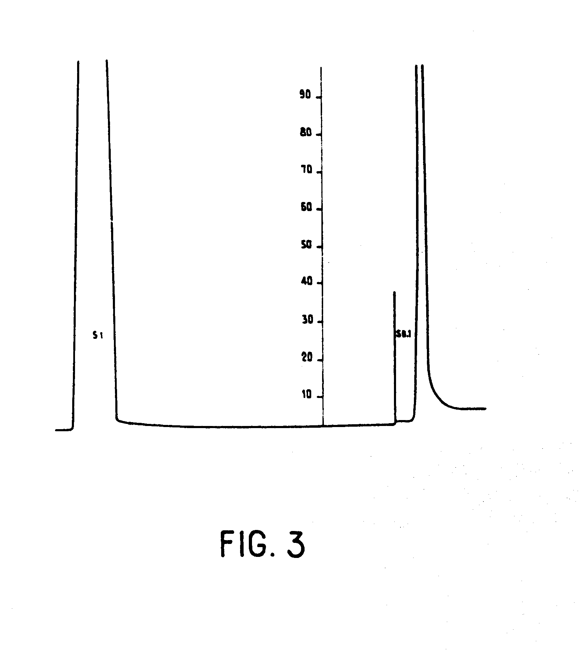

Sur les figures ci-annexées :
- la figure 1 représente les immunoélectrophorèses quantitatives linaires de type "line-absorption" sur Neisseria
  gonorrhoeae pour les sérotypes :
  A figure 1a
  B figure 1b
- la figure 2 représente des immunoélectrophorèses comme

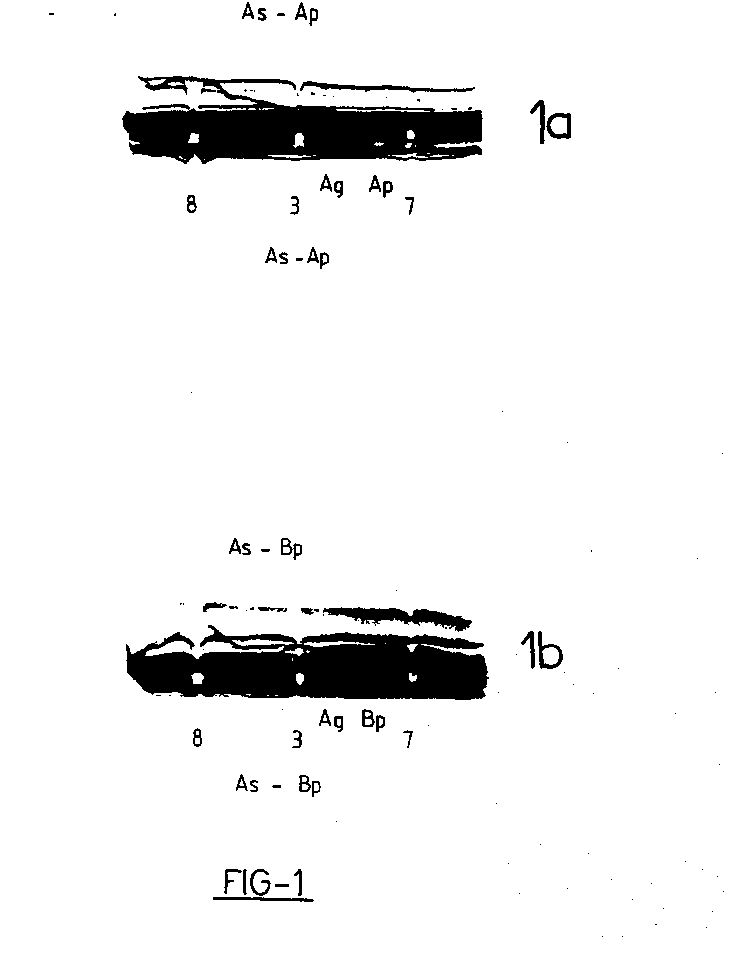

  la figure 1 mais pour les sérotypes :
  C figure 2a
  D figure 2b
- la figure 3 représente une chromatographie d'affinité
  d'un extrait protéique de Neisseria gonorrhoeae.

Exemple 1 :

        Cet exemple a pour objet la mise en évidence des
lectines-facteurs d'attachement décrits précédemment. Les
sept sucres suivants ont été testés : maltose, galactosylamine,fucosylamine, glucosamine, acetylglucosamine, D galactosamine, thiogalactoside. Ces sucres ont été fixés sur
des billes d'agarose par des techniques connues de l'homme
de métier. Pour la mise en oeuvre de l'exemple proprement
dit une suspension bactérienne de 0,5 ml a été mise en
contact avec 0,5 ml de sucre immobilisé sur des billes.
Après incubation, les billes ont été lavées puis colorées
et observées au microscope. Suivant les sucres, deux cas
se sont présentés. Pour les billes recouvertes d'α-D glucosamine ou de maltose, Neisseria gonorrhoeae adhérait sur
ces billes. Par contre, pour les autres sucres, aucune
adhésion du gonocoque n'était observée. Afin de mettre en
évidence la spécificité de ces fixations, la possibilité
d'inhiber la fixation du gonocoque a été étudiée en faisant
incuber préalablement la suspension de Neisseria gonorrhoeae
avec un sucre libre correspondant au sucre fixé mis en oeuvre dans l'étape suivante. Les résultats observés avec des souches de référence de deux collections et des souches
sauvages sont identiques et mettent en évidence la fixation
tion spécifique de ces lectines-facteurs d'attachement.
La fixation était en effet inhibée uniquement si le sucre
libre correspondant au mélange d'incubation étant ajouté
en concentration suffisante (25 mg/ml). L'inhibition n'était
tait réalisée avec aucun autre sucre. Donc, il existe à
la surface du gonocoque des lectines-facteurs d'attachement
qui permettent l'adhésion sur des récepteurs immobilisés.
Ceux-ci possèdent entre autres des résidus glucosamine et
maltose. Ceci permet la purification de ces lectines-facteurs par chromatographie d'affinité sur des récepteurs
sucrés immobilisés.

Exemple 2 :

        Les extraits antigéniques pariétaux solubilisés du
gonocoque ont été soumis à une immunoélectrophorèse type
"line-absorption". Les mélanges antigéniques étaient incorporés à des bandes d'agarose, ils migraient dans les gels
contenant les antisérums polyvalents correspondants et quand
un antigène rencontrait son anticorps spécifique à la concentration appropriée du point d'équivalence, une ligne de
précipitation était obtenue. Les figures 1 et 2 schématisent l'interprétation des résultats observés. Dans ces
immunoélectrophorèses, les antigènes (Ag) étaient constitués
par des extraits pariétaux (p) solubilisés de quatre souches
de référence de gonocoque (GEIZER A, B, C, D), les anticorps
(As) correspondaient aux antisérums spécifiques des extraits
pariétaux. Les puits contenaient les sucres immobilisés suivants : puits 3 : glucosamine, puits 7 : galactosamine,
puits 8 : maltose. Pour les quatre sérotypes de GEIZER testés, les résultats obtenus sont identiques : absorption
des lignes de précipitation avec les sucres immobilisés
maltose glucosamine uniquement.

Exemple 3 :

Cet exemple montre la purification des facteurs d'attachement de Neisseria gonorrhoeae sérotype C de GEIZER réalisé par chromatographie d'affinité sur colonne agarose-glucosamine. Cette purification a été effectuée à partir de l'extrait protéique pariétal solubilisé du gonocoque : après fixation des lectines-facteurs d'attachement sur les récepteurs sucrés immobilisés et élimination dans le premier pic de la figure 3 des protéines n'interréagissant pas avec le sucre fixé, ces lectines-facteurs d'attachement étaient éluées par une solution de glucosamine à 0,25 M dans le deuxième pic. Ces lectines-facteurs d'attachement permettent de proposer :

. des explications concernant les interactions hôte-gonocoque au niveau des cellules épithéliales de l'urètre et du rhinopharynx,

. des thérapeutiques curatives mais surtout préventives en raison des propriétés antigéniques de ces facteurs d'attachement,

. des techniques nouvelles de diagnostic des infections gonococciques.

REVENDICATIONS

1. Lectines-facteurs d'attachement de la membrane externe de Neisseria gonorrhoeae autres que les protéines majeures (protéine II par exemple).

2. Lectines-facteurs d'attachement de Neisseria gonorrhoeae, selon la revendication 1, qui sont fixées sur des récepteurs sucrés tels que glucosamine ou maltose.

3. Récepteurs d'attachement spécifiques des lectines-facteurs d'attachement selon l'une des revendications 1 et 2.

4. Anticorps dressés contre les lectines-facteurs d'attachement selon l'une des revendications 1 et 2.

5. Procédé de préparation des lectines-facteurs d'attachement selon l'une des revendications 1 et 2, caractérisé en ce que :

a) on prépare à partir d'une souche de gonocoque un extrait antigénique pariétal, solubilisé ultérieurement,

b) on met cet extrait en présence d'un récepteur sucré immobilisé,

c) puis on sépare les lectines-facteurs d'attachement fixées sur ledit récepteur.

6. Procédé selon la revendication 5, caractérisé en ce que le récepteur sucré immobilisé comporte au moins un sucre tel que la glucosamine et le maltose.

7. Procédé selon la revendication 6, caractérisé en ce que le sucre est choisi parmi l'α-D-glucosamine ou le maltose.

8. Procédé selon l'une des revendications 5 à 7, caractérisé en ce que le support immobilisant le sucre est l'agarose.

9. Composition pharmaceutique contenant à titre de principe actif au moins une lectine-facteur d'attachement ou un récepteur ou leurs analogues spécifiques ou un anticorps selon l'une des revendications 1 à 4.

10. Procédé de mise en évidence et/ou de dosage des gonocoques dans un milieu, caractérisé en ce qu'il comporte au moins une étape de fixation des lectines-facteurs d'attachement des gonocoques sur un sucre.

11. Procédé selon la revendication 10 caractérisé en ce que le sucre est fixé sur un support.

12. Procédé selon la revendication 11, caractérisé en ce que le support est un support d'agarose.

13. Procédé selon l'une des revendications 10 à 12, caractérisé en ce que le procédé mis en oeuvre est un procédé de dosage immunologique.

14. Procédé selon la revendication 13, caractérisé en ce que le procédé mis en oeuvre comporte une étape d'immunoélectrophorèse.

FIG-1

As - Cp

2a

Ag Cp

8          3          7

As -Cp

As - Dp

2b

As -Dp

FIG-2

FIG. 3

**Office européen
des brevets**

**RAPPORT DE RECHERCHE EUROPEENNE**

**0214057**

Numéro de la demande

EP    86  40  1916

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | BIOLOGICAL ABSTRACTS, vol. 75, no. 2, 1983, résumé no. 11571, Philadelphia, P.A., US; E.R. GUBISH Jr. et al.: "Attachment of gonococcal pill to lectin-resistant clones of Chinese hamster ovary cells", & INFECT. IMMUN. 37(1): 189-194, 1982 | | A 61 K  37/02<br>A 61 K  39/095<br>A 61 K  39/40<br>G 01 N  33/571<br>G 01 N  33/548<br>G 01 N  33/561 |
| | --- | | |
| A | CHEMICAL ABSTRACTS, vol. 103, no. 21, 25 novembre 1985, page 553, résumé no. 176837n, Columbus, Ohio, US; R.F. REST et al.: "Stimulation of human leukocytes by protein II+ gonococci is mediated by lectin-like gonococcal components", & INFECT. IMMUN. 1985, 50(1), 116-22 | | |
| | | | **DOMAINES TECHNIQUES RECHERCHES (Int. Cl 4)** |
| | --- | | |
| A | CHEMICAL ABSTRACTS, vol. 85, no. 3, 19 juillet 1976, page 449, résumé no. 18383s, Columbus, Ohio, US; A.N. JAMES et al.: "Attachment of gonococci to sperm. Influence of physical and chemical factors", & BR. J. VENER. DIS. 1976, 52(2), 128-35 | | G 01 N<br>A 61 K |
| | ----- | | |

Le present rapport de recherche a été établi pour toutes les revendications

| Lieu de la recherche | Date d'achèvement de la recherche | Examinateur |
|---|---|---|
| LA HAYE | 27-10-1986 | GRIFFITH G. |